# EUROPEAN PATENT SPECIFICATION

(11) **EP 1 852 060 B1**
(45) Date of publication and mention of the grant of the patent: **18.12.2013**
(21) Application number: 07290544.1
(22) Date of filing: 30.04.2007
(51) Int. Cl.: A61B 5/00, G06F 19/00

(54) **Method and apparatus for displaying labour related information associated to an obstetrics patient**
Verfahren und Vorrichtung zur Anzeige von wehenbezogenen Informationen in Zusammenhang mit einem obstetrischen Patienten
Procédé et appareil pour l'affichage d'informations liées au travail chez un patient en obstétrique

(30) Priority: 01.05.2006 US 416281; 01.05.2006 CA 2545339
(43) Date of publication of application: 07.11.2007
(73) Proprietor: LMS Medical Systems, Ltd., Montreal, Quebec H4A 3S5 (CA)
(72) Inventor: Hamilton, Emily, Verdun Québec H3E 1K8 (CA)
(74) Representative: Blot, Philippe Robert Emile

(56) References cited:
- EP-A- 0 808 603
- WO-A-2004/041059
- US-A1- 2005 049 509
- US-B2- 6 907 284

## Description

### FIELD OF THE INVENTION

The present invention relates generally to the field of obstetrics, and more specifically to a method and apparatus for monitoring labor progression and for providing a user interface to display data conveying maternal and fetal information during labor.

### BACKGROUND

Birth related injuries are rare but devastating events because the consequences can lead to lifelong impairment for the baby, family and society in general. During labor, clinical staff monitors various health characteristics of the obstetrics patients in order to obtain a qualitative assessment of the mother's and the fetus's well-being. The clinical staff uses visual methods to study the heart rate tracings of the foetus and mother and the measurements and relies on their experience to deduce the degree of maternal and fetal well being and to assess whether labour is progressing normally. Low incidence-high consequence injuries are particularly challenging to prevent. Because of their rarity, it is difficult for clinicians to remain vigilant, to develop substantial experience with them and to maintain the appropriate skills. Over-caution can result in a high rate of unnecessary interventions in the majority of patients who are not at risk for the rare adverse event.

In addition, several working conditions known to promote error in judgement are prevalent in obstetrics. Fatigue, a well-known error producing condition, is common. Frequent false alarms, because the warning signs are numerous and non-specific, lead to complacency and missed recognition of a true problem. Workload is unpredictable and heavy demands may divert the teams focus from a developing problem. The required assessments are numerous and complex. Clinicians must integrate many pieces of information pertaining to both the mother and the baby's conditions, over prolonged periods of time, communicate their assessments clearly among team members and implement corrective measures before it is too late. Moreover, the conditions present in one birth may be quite different from another such that a particular observation may be normal under one set of circumstances and abnormal for another.

European Patent Application No. 0 808 603 published on November 26, 1997 and entitled "Fetal monitoring system and method", hereinafter EP'603, describes amongst others, a fetal monitoring system allowing for the simultaneous monitoring of more than one fetal monitor. EP'603 describes the use of a display including a plurality of viewing windows, where each viewing window displays labour related information associated with a respective "bed" (obstetrics patient). In EP'603, the user of the system may select one of the viewing windows so that information associated with the corresponding patient is displayed on a full-screen.

Although many metrics have been developed, a deficiency associated with existing approaches to labour monitoring is that they do not provide suitable functionality for allowing the clinical staff to effectively manage and make use of the large amount of information gathered during labour progression. As such, the clinical staff is frequently overloaded with information. This overload of information often obscures the more crucial facts and makes critical information difficult to discern. To date, there exists no suitable tool or system for allowing the clinical staff to view a concise representation of labour progression for an obstetrics patient.

In the context of the above, there is a need in the industry to provide a method and device for displaying data conveying labor related information for an obstetrics patient that alleviates at least in part problems associated with the existing methods and devices.

### SUMMARY

The invention is defined in the independent claims.

In accordance with an aspect, the invention provides a method for displaying labour related information. The method comprises providing a set of possible viewing windows, each viewing window in the set of possible viewing windows conveying a feature measurement related to labour progression. A certain viewing window in the set of possible viewing windows conveys a given feature measurement and a safety limit associated to the given feature measurement. The method further comprises displaying a first viewing window selected from the set of possible viewing windows. The method further comprises displaying at least one control allowing a user to select a subset of viewing windows from the set of possible viewing windows, the subset of viewing windows including at least one viewing window other than the first viewing window. The method further comprises displaying the selected subset of viewing windows simultaneously with the first viewing window.

In accordance with another aspect, the invention provides a computer readable storage medium storing a program element suitable for execution by a CPU. The program element implements the above-described method for displaying labour related information.

In accordance with another aspect, the invention provides a server system storing a program element suitable for execution by a CPU. The program element implements the above-described method for displaying labour related information.

In accordance with another aspect, the invention provides an apparatus implementing a graphical user interface for displaying labour related information. The apparatus comprises input means for receiving signals conveying labour information and processing means in communication with the input means. The processing means are adapted for implementing the above-described method.

In accordance with another aspect, the invention provides a labour monitoring system. The labour monitoring system comprises a first sensor for receiving a signal indicative of a fetal heart rate and a second sensor for receiving a uterine contraction signal associated with an obstetrics patient. The labour monitoring system also comprises an apparatus implementing a graphical user interface module for displaying labour related information. The apparatus comprises first input means in communication with the first sensor for receiving the fetal heart rate signal and second means in communication with the second sensor for receiving the uterine contraction signal. The apparatus as comprises processing means in communication with the first and second input means. The processing means are adapted for implementing the above-described method. The labour monitoring system also comprises display means in communication with the apparatus.

In accordance with another broad aspect, the invention provides a client-server system implementing a graphical user interface for displaying for displaying labour related information. The client-server system comprises a client system and a server system operative to exchange messages over a data network. The server system stores a program element for execution by a CPU, the program element implementing the above-described method.

In accordance with another aspect, the invention provides a computer readable storage medium storing a program element suitable for execution by a CPU where the program element implements a graphical user interface module for displaying labour related information. The graphical user interface module is adapted for displaying a first viewing window selected from a set of possible viewing windows, each viewing window in the set of possible viewing windows conveying a feature measurement related to labour progression. At least one viewing window in the set of possible viewing windows conveys a given feature measurement and a safety limit associated to the given feature measurement. The graphical user interface module is adapted for displaying at least one control allowing a user to select a subset of viewing windows from the set of possible viewing windows, the subset of viewing windows including at least one viewing window other than the first viewing window. The graphical user interface module is adapted for displaying the selected subset of viewing windows simultaneously with the first viewing window. In response to the given feature measurement exceeding the safety limit associated to the given feature measurement, the graphical user interface module is adapted for displaying information to attract the attention of the user to the viewing window conveying the given feature measurement.

An advantage of the above described broad aspect of the present invention is that it allows a user to view simultaneously multiple information elements related to labor progression thereby increasing the clinical staff's situational awareness by allowing the clinical staff to assess more consistently how labour is progressing by taking into account multiple information elements. In addition, but providing a safety limit for a feature measurement, the user can readily observe whether the feature is within or exceed the safety limit.

Another advantage of the above described aspect of the present invention allows a user to select which information elements to view for a given obstetrics patient. This provides the user with flexibility regarding what is being observed such that information elements considered to be more important by the user may be displayed and those considered of lesser importance may be concealed. As such, the clinical staff can view a concise representation of labor related information for an obstetrics patient.

In accordance with a specific implementation, the safety limit associated to the given feature measurement includes at least one threshold value.

In a first specific implementation, the graphical user interface module displays a visual indicator in association with the viewing window conveying the given feature measurement when the given feature measurement exceeds the at least one threshold value. The visual indicator may be represented in any suitable manner. In a specific implementation, the threshold value corresponds to a boundary value set by best practices or by a healthcare establishment (eg. hospital) policy.

In a second specific implementation the graphical user interface module is adapted to display the viewing window conveying the given feature measurement when the given feature measurement exceeds the at least one threshold value. The at least one threshold value may be conveyed in textual or graphical form in the associated viewing window.

In accordance with a specific implementation, the control allows the user to select the subset of the set of windows by using an input device selected from the set consisting of a mouse, keyboard, pointing device, speech recognition unit and touch sensitive screen. In a specific implementation the control includes a selection box. In an alternative implementation, the graphical user interface module is adapted for displaying a set of controls, each control in said set of controls being associated to a respective viewing window in the set of possible viewing windows, each control allowing a user to select an associated viewing window.

In accordance with another aspect, the invention provides an apparatus for implementing a user interface for displaying labour related information. The apparatus comprises an input for receiving signals conveying labour information and a processing unit in communication with the input. The processing unit is operative for implementing a graphical user interface module for displaying labour related information. The graphical user interface module is adapted for displaying a first viewing window selected from a set of possible viewing windows. Each viewing window in the set of possible viewing windows conveys a feature measurement related to labour progression derived at least in part from the signals received at the input. At least one viewing window in the set of possible viewing windows conveys a given feature measurement and a safety limit associated to the given feature measurement. The graphical user interface module is also adapted for displaying at least one control allowing a user to select a subset of viewing windows from the set of possible viewing windows, the subset of viewing windows including at least one viewing window other than the first viewing window. The graphical user interface module is also adapted for displaying the selected subset of additional viewing windows simultaneously with the first viewing window. In response to the given feature measurement exceeding the safety limit associated to the given feature measurement, the graphical user interface module is also adapted for displaying information to attract the attention of the user to the viewing window conveying the given feature measurement. The apparatus also includes an output in communication with the processing unit suitable for releasing a signal for causing a display unit to display the graphical user interface module.

In accordance with another aspect, the invention provides a method for displaying labour related information. The method comprises receiving signals conveying labour information. The method also comprises displaying a first viewing window selected from a set of possible viewing windows, each viewing window in the set of possible viewing windows conveying a feature measurement related to labour progression derived at least in part from the signals received. At least one viewing window in the set of possible viewing windows conveys a given feature measurement and a safety limit associated to the given feature measurement. The method also comprises displaying at least one control allowing a user to select a subset of viewing windows from the set of possible viewing windows, the subset of viewing windows including at least one viewing window other than the first viewing window. The method also comprises displaying the selected subset of viewing windows simultaneously with the first viewing window. The method also comprises displaying information to attract the attention of the user to the viewing window conveying the given feature measurement in response to the given feature measurement exceeding the safety limit associated to the given feature measurement.

In accordance with another aspect, the invention provides a labour monitoring system comprising a first sensor for receiving a signal indicative of a fetal heart rate, a second sensor for receiving a uterine contraction signal associated with an obstetrics patient, an apparatus for implementing a user interface for displaying labour related information and a display unit. The apparatus includes a first input in communication with the first sensor for receiving the fetal heart rate signal. The apparatus also includes a second input in communication with the second sensor for receiving the uterine contraction signal. The apparatus also includes a processing unit in communication with the first input and the second input and operative for implementing a graphical user interface module for displaying labour related information. The graphical user interface module is adapted for displaying a first viewing window selected from a set of possible viewing windows. Each viewing window in the set of possible viewing windows conveys a feature measurement related to labour progression derived at least in part from the signals received at the first input and the second input. At least one viewing window in the set of possible viewing windows conveys a given feature measurement and a safety limit associated to the given feature measurement. The graphical user interface module is also adapted for displaying at least one control allowing a user to select a subset of viewing windows from the set of possible viewing windows, the subset of viewing windows including at least one viewing window other than the first viewing window. The graphical user interface module is also adapted for displaying the selected subset of additional viewing windows simultaneously with the first viewing window. In response to the given feature measurement exceeding the safety limit associated to the given feature measurement, the graphical user interface module is adapted for displaying information to attract the attention of the user to the viewing window conveying the given feature measurement. The apparatus also includes an output in communication with the processing unit, wherein the output is suitable for releasing an output signal for causing a display unit to display the graphical user interface module. The display unit of the labour monitoring system is in communication with the output of the apparatus and is responsive to the output signal to display the graphical user interface module.

In accordance with another aspect, the invention provides a client system adapted for communication with a server system for implementing a graphical user interface module for displaying labour related information for an obstetrics patient. The client system and the server system are operative to exchange messages over a data network. The client system is adapted for receiving from the server system a program element for causing a graphical user interface module to be displayed at the client system. The graphical user interface module is adapted for displaying a first viewing window selected from a set of possible viewing windows, each viewing window in the set of possible viewing windows conveying a feature measurement related to labour progression. At least one viewing window in the set of possible viewing windows conveys a given feature measurement and a safety limit associated to the given feature measurement. The graphical user interface module is also adapted for displaying at least one control allowing a user to select a subset of viewing windows from the set of possible viewing windows, the subset of viewing windows including at least one viewing window other than the first viewing window. The client system is adapted for transmitting data indicative of the selected subset of the set of possible viewing windows to the server system. The client system is also adapted for receiving from the server system instructions for causing the selected subset of viewing windows to be displayed simultaneously with the first viewing window. The client system is also adapted for receiving from the server system instructions for causing information to be displayed to attract the attention of the user to the viewing window conveying the given feature measurement in response to the given feature measurement exceeding the safety limit associated to the given feature measurement.

In accordance with specific implementations the data network may be of any suitable network configuration including Intranets and the Internet.

In accordance with another aspect, the invention provides an apparatus for implementing a user interface for displaying labour related information. The apparatus comprises means for receiving signals conveying labour information. The apparatus also comprises means for implementing a graphical user interface module for displaying labour related information. The graphical user interface module is adapted for displaying a first viewing window selected from a set of possible viewing windows. Each viewing window in the set of possible viewing windows conveys a feature measurement related to labour progression derived at least in part from the signals received. At least one viewing window in the set of possible viewing windows conveys a given feature measurement and a safety limit associated to the given feature measurement. The graphical user interface module is also adapted for displaying at least one control allowing a user to select a subset of viewing windows from the set of possible viewing windows, the subset of viewing windows including at least one viewing window other than the first viewing window. The graphical user interface module is also adapted for displaying the selected subset of additional viewing windows simultaneously with the first viewing window. In response to the given feature measurement exceeding the safety limit associated to the given feature measurement, the graphical user interface module displays information to attract the attention of the user to the viewing window conveying the given feature measurement. The apparatus also comprises means for releasing a signal for causing a display unit to display the graphical user interface module.

In accordance with yet another aspect, the invention provides a computer readable storage medium storing a program element suitable for execution by a CPU, the program element implementing a graphical user interface module for displaying labour related information. The graphical user interface module is adapted for displaying a first viewing window selected from a set of possible viewing windows, each viewing window in the set of possible viewing windows conveying a feature measurement related to labour progression. The set of possible viewing windows includes at least one grouping having at least two viewing windows. The graphical user interface module is also adapted for displaying at least one control allowing a user to select a subset of viewing windows from the set of possible viewing windows and for displaying the selected subset of viewing windows simultaneously with the first viewing windows.

In accordance with yet another aspect, the invention provides a computer readable storage medium storing a program element suitable for execution by a CPU, the program element implementing a graphical user interface module for displaying labour related information. The graphical user interface module is adapted for receiving a signal conveying a user identifier associated to a user of the system. The graphical user interface module is also adapted for displaying a first viewing window selected from a set of possible viewing windows, each viewing window in the set of possible viewing windows conveying a feature measurement related to labour progression. The graphical user interface module is also adapted for displaying at least one additional viewing window other than the first viewing window. The additional viewing window is selected from the set of possible viewing windows at least in part on the basis of the signal conveying the user identifier. The graphical user interface module is also adapted for displaying at least one control allowing a user to select a subset of viewing windows from the set of possible viewing windows and for displaying the selected subset of viewing windows simultaneously with the first viewing window and with the at least one additional viewing window.

These and other aspects and features of the present invention will now become apparent to those of ordinary skill in the art upon review of the following description of specific embodiments of the invention in conjunction with the accompanying drawings.

### BRIEF DESCRIPTION OF THE DRAWINGS

In the accompanying drawings:
Fig. 1 shows a high-level functional block diagram of a system including a processing unit for monitoring labour for an obstetrics patient in accordance with a specific example of implementation of the present invention;
Fig. 2 shows a block diagram of the processing unit depicted in figure 1 in accordance with a specific example of implementation of the present invention;
Fig. 3a shows a specific example of implementation of a graphical user interface implemented by the system shown in figure 1 for providing labour related information in accordance with a first non-limiting example of implementation of the invention;
Fig. 3b shows a specific example of implementation of a graphical user interface implemented by the system shown in figure 1 for providing labour related information in accordance with a second non-limiting example of implementation of the invention;
Fig. 3c shows a specific example of implementation of a graphical user interface implemented by the system shown in figure 1 for providing labour related information in accordance with a third non-limiting example of implementation of the invention;
Fig. 4a and 4b show examples of controls allowing a user to select a subset of information elements in accordance with specific examples of implementation of the invention;
Fig. 5 is a block diagram of an apparatus for providing labour related information in accordance with a specific example of implementation of the present invention;
Fig. 6 is a high level conceptual block diagram of a program element for implementing a graphical user interfaces of the type shown in figures 3a, 3b and 3c in accordance with a specific example of implementation of the present invention;
Fig. 7 shows a functional block diagram of a client-server system for providing labour related information in accordance in accordance with an alternative specific non-limiting example of implementation of the present invention.

Other aspects and features of the present invention will become apparent to those ordinarily skilled in the art upon review of the following description of specific embodiments of the invention in conjunction with the accompanying figures.

### DETAILED DESCRIPTION

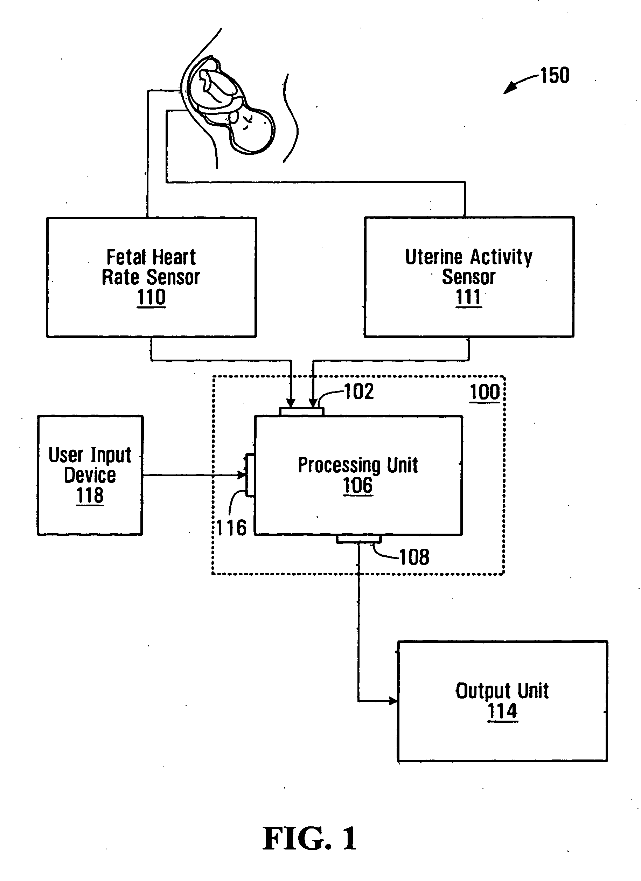

With reference to Fig. 1, there is shown a configuration of a system 150 for monitoring labor progress for an obstetrics patient comprising a fetal heart rate sensor 110, a uterine activity sensor 111, a user input device 118, an apparatus 100 implementing a user interface for displaying labour related information and an output unit 114.

The user input device 118 is for receiving data from a user of the system. The data may convey commands directed to controlling various features of the user interface implemented by apparatus 100 and, optionally, may also convey various measurements associated with the obstetrics patients taken during labour. The type of data received through input device 118 may vary depending on the type of information that the apparatus 100 is adapted to process and interpret. In a specific example of implementation, input device 118 allows a user to enter a selection regarding additional viewing windows to be displayed on the graphical user interface implemented by apparatus 100. Optionally, input device 118 allows a user to a user identifier associated to the user. Other specific examples of the type of information that may be provided through input device 118 will be described later on in the specification. The user input device 118 includes any one or a combination of the following: keyboard, pointing device, touch sensitive surface, actuator/selection switches or speech recognition unit.

The fetal heart rate sensor 110 is for detecting a fetal heart rate of a fetus in-utero, also referred to as a fetus in the womb. The fetal heart rate sensor 110 samples the fetal heart rate at a certain pre-determined frequency to generate the signal indicative of the fetal heart rate. Fetal heart rate sensors are well known in the art to which this invention pertains and any suitable sensor for detecting a fetal heart rate may be used and as such will not be described further here.

The uterine activity sensor 111 is for monitoring uterine activity (TOCO). The sensor samples the contraction pattern at a certain pre-determined frequency to generate the signal indicative of uterine activity. Sensors for monitoring uterine activity are well known in the art to which this invention pertains and any suitable sensor may be used and as such will not be described further here.

Optionally, the fetal monitoring system 150 may include other sensors (not shown) for measuring labour progress and the fetus' tolerance to labour. Such sensors may include for example:
- a sensor for measuring the maternal oxygen saturation
- a sensor for measuring the foetal oxygen saturation
- a sensor for measuring maternal blood pressure

Suitable sensors other than the ones described above may be used.

The output unit 114 is coupled to the apparatus 100 and receives a signal causing the output unit 114 to display a graphical user interface module implemented by apparatus 100. The output unit 114 may be in the form of a display screen, a printer or any other suitable device for conveying to the physician or other health care professional the progression of labour related information. In a non-limiting implementation, the output unit 114 includes one or more display monitors to display the graphical user interface. The output unit 114 may also include a printer device for providing a paper print out of the graphical user interface implemented by apparatus 100.

The apparatus 100 includes a first input 102, a second input 116, a processing unit 106 and an output 108. The first input 102 is for receiving signals conveying labour information. In the example depicted the first input is for receiving a fetal heart rate signal from the fetal heart rate sensor 110 and the uterine activity signal from the uterine activity sensor 111. It will be readily appreciated that, although the fetal heart rate signal and the uterine activity signal are received at a same input 102 in the embodiment illustrated in the figures, the signals may be provided to processing unit 106 through separate inputs. The second input 116 is for receiving data from a user through input device 118. The processing unit 106 processes the signals received at input 102 as well as the user data received at input 116 and to derive various information elements related to labour progression. The processing unit 106 also implements a graphical user interface module for displaying labour related information associated to the obstetrics patient. The output 108 is for releasing a signal for causing output unit 114 to display the graphical user interface module implemented by processing unit 106.

### Processing unit 106

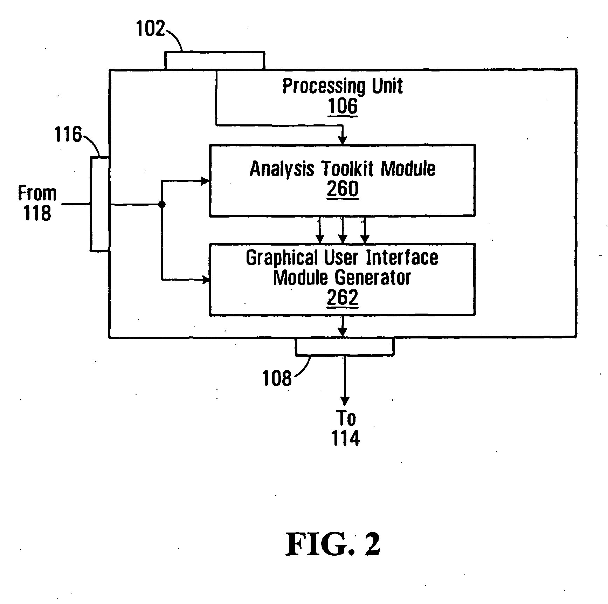

A specific example of implementation of the processing unit 106 of apparatus 100 is depicted in greater detail in figure 2 of the drawings. As shown, the processing unit 106 includes two sub-components namely an analysis toolkit module 260 and a graphical user interface module generator 262.

The analysis toolkit module 260 includes a set of processing entities adapted for receiving data from the fetal heart rate sensor 110, the uterine activity sensor 111 and the input device 118 and processing that data to derive therefrom various information elements related to labour progression.

In a non-limiting implementation, the analysis toolkit module 260 may be viewed as a grouping of analysis tools, each tool being operative for deriving one or more information elements related to labour progression. Examples of the types of tools that may be part of the analysis toolkit module 260 are described below. In addition, certain embodiments of the invention may be configured such that the analysis toolkit module 260 is adapted to be augmented with additional tools. Such a modular configuration is particularly advantageous as it allows the new tools to be added as new developments arise. Such a modular configuration is also advantageous as it allows the end user to customise the system 150 (figure 1) so that it includes the desired tools and conveys the desired information.

In a specific example of implementation, the analysis toolkit module 260 includes a tool for deriving over time levels of risk associated with the labour progression. Any suitable tool for deriving a level of risk may be used. In a very specific example, the level of risk associated with labour progression is derived on the basis of method described in U.S. Patent Application Serial No. 10/113,788 filed on April 1, 2002 and presently pending and assigned to LMS Medical Systems Ltd. In this document a method for monitoring the condition of a fetus to assess a degree of risk of developing a permanent neurological condition is described. The degree of risk of developing a permanent neurological condition is expressed as a likelihood that the condition of the fetus belongs to a class in a group of classes, where each class in the group of classes is associated with a pre-defined fetal condition. Other methods for deriving a level of risk associated with labour progression may be used. The tool for deriving levels of risk associated with the labour progression releases these information elements over time for transmission to the graphical user interface module generator 262.

In a specific example of implementation, the analysis toolkit module 260 includes a tool for deriving an expected cervical dilation over time. In a non-limiting example of implementation, the expected dilation of the cervix over time is derived on the basis of method described in U.S. Patent No. 6,423,016 issued July 23, 2002 and presently assigned to LMS Medical Systems Ltd. In this document, a method for tracking the labor progress of a patient during childbirth is described for deriving an expected cervical dilation on the basis of a group of clinical measurements associated to a patient. The group of clinical measurements includes data elements indicative of a measurement of a previous dilation of the cervix of the patient, a contraction count, a previous level of descent of the child, a previous effacement measurement of the cervix, an epidural status and a parity status. The actual observed cervical dilations were plotted over time on a graph against a range of expected cervical dilation derived on the basis of the mathematical model which allows the clinical staff to assess whether the cervix was dilating in accordance with an expected progression as described by the model. The group of clinical measurements described above may be provided through input device 118 shown in figure 1 of the drawings. Other methods for deriving an expected cervical dilation may be used. The tool for deriving expected cervical dilations releases these information elements over time for transmission to the graphical user interface module generator 262.

In a specific example of implementation, the analysis toolkit module 260 includes a tool for deriving fetal heart rate feature measurements. Fetal heart rate feature measurements include, for example, mean baseline, mean baseline variability, decelerations and accelerations amongst others. Any suitable tool for deriving fetal heart rate feature measurements may be used. The tool for deriving fetal heart rate feature measurements releases these measurements over time for transmission to the graphical user interface module generator 262.

In a specific example of implementation, the analysis toolkit module 260 also includes a tool for deriving a running average of a uterine contraction count associated with the obstetrics patient. More specifically the tool processes the signal received from the uterine activity sensor 111 to compute the running average of a uterine contraction count. Any suitable method for the compute the running average may be used. The tool for deriving the running average of the uterine contraction count releases these measurements over time for transmission to the graphical user interface module generator 262. Optionally, this tool also releases a data element indicative of a threshold average uterine contraction count. The threshold average contraction count may be set in accordance best practices and/or in accordance with hospital/care-giver facility policy.

In a specific example of implementation, the analysis toolkit module 260 includes a tool for allowing a user to select a portion of the fetal heart rate signal for display in a zoomed in fashion. A non-limiting example of such a tool is described in U.S. Patent No. 6,907,284, entitled "Method and Apparatus for Displaying a Heart Rate Signal", which issued June 14, 2005 and is presently assigned to LMS Medical Systems Ltd. The tool for allowing a user to select a portion of the fetal heart rate signal for display in a zoomed in fashion releases data over time for transmission to the graphical user interface module generator 262.

In a specific implementation, at least some of the tools are adapted for generating information element indicating a safety limit for the metrics being measured and/or observed. The safety limit marks a boundary between measurements/observations considered to be safe or "normal" and measurements/observations considered as being associated to riskier situations. It will be readily apparent that multiple safety limits may be used for marking boundaries between different levels of risk (eg. low, intermediate, high etc...). The number of safety limits for a given measurement will typically depend on the type of measurement being observed. The safety limit(s) may be set in accordance best practices and/or in accordance with hospital/care-giver facility policy. The manner in which the safety limits are selected and well as the number of safety limits is not critical to the invention and as such will not be described further here.

It will be readily apparent that additional tools may be included in, and certain tools omitted from, the analysis toolkit module 260.

The graphical user interface module generator 262 will now be described in greater detail herein below.

### Graphical Interface Module Generator 262

The graphical user interface module generator 262 is in communication with the analysis toolkit module 260 and receives therefrom over time a plurality of information elements related to labour progression. The graphical user interface module 262 also receives data indicative of user selection(s). The data may be directly received through input 116 or may be relayed through the analysis toolkit module 260.

The graphical user interface module generator 262 processes the plurality of information elements related to labour progression and the data indicative of user selection(s) to generate a graphical user interface module.

More specifically, the graphical user interface module generator 262 is adapted to generate a signal for displaying a first viewing window selected from a set of possible viewing windows. Each viewing window in the set of possible viewing windows conveys a feature measurement related to labour progression. One or more of the viewing windows in the set of possible viewing windows convey a given feature measurement and an associated safety limit. The graphical user interface module generator 262 generates a signal for displaying one or more controls allowing a user to select a subset of viewing windows from the set of possible viewing windows, the subset of viewing windows including at least one viewing window other than the first viewing window. In addition, the graphical user interface module generator 262 processes the plurality of information elements related to labour progression and the data indicative of user selection(s) to generate a signal for displaying a set of additional viewing windows as part of the graphical user interface module simultaneously with the first viewing window. In a specific implementation each additional viewing window is adapted to display information conveying one or more information elements related to labour progression. In the additional viewing windows, when the information elements vary over time, such information elements are preferably conveyed as tracings over time. The specific appearance of the graphical user interface module may vary from one implementation to the other. For example, the positions and general layout of the various viewing windows may be different from one implementation to the other. Also, certain implementations may show various components of the user interface module on different display screens instead of a same display screen.

In a first embodiment, the graphical user interface module generator 262 generates a signal, in response to a given feature measurement exceeding its associated safety limit, for displaying information to attract the attention of the user to a viewing window in the set of possible viewing windows conveying the given feature measurement.

In a second embodiment, the set of possible viewing windows includes at least one grouping, the at least one grouping including at least two viewing windows for allowing the user to select two (2) or more viewing windows simultaneously.

In a third embodiment, the graphical user interface module generator 262 generates a signal for displaying at least one additional viewing window other than the first viewing window selected from the set of possible viewing windows at least in part on the basis a user identifier.

In a specific implementation, the graphical user interface module generator 262 may be viewed as a group of display modules, each display modules in the group being associated to a corresponding one of the analysis tools in the analysis toolkit module 260. Each display module is adapted to generate a respective graphical representation in the form of viewing window of the information generated by the respective analysis tools. The graphical user interface module generator 262 also includes a consolidation module in communication with the display modules in the group of display modules for generating the graphical user interface.

Below are described specific examples of implementation of the graphical user interface module. The person skilled in the art, in light of the present specification, will readily appreciate that many variants are possible.

### First Example

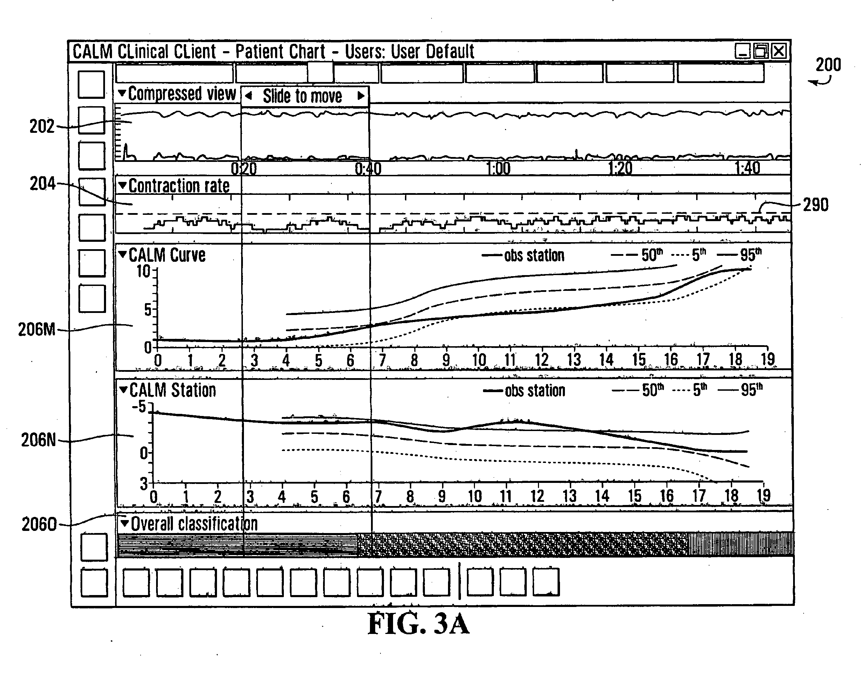

With reference to figure 3a, a first specific example of a graphical user interface module 200 implemented by graphical user interface module generator 262 (shown in figure 2) is shown. The graphical user interface module 200 includes a first viewing window 202 and a second viewing window 204. In the first viewing window 202, a first tracing indicative of a fetal heart rate signal is displayed and in the second viewing window 204, a second tracing indicative of a uterine contraction pattern associated with the obstetrics patient is displayed. The graphical user interface module also includes a set of controls 206m-n-o for allowing a user to select for display additional viewing windows from a set of possible viewing windows. As described above, the additional viewing windows in the set of possible viewing windows are adapted for conveying various information elements related to the assessment of labour for the obstetrics patient. Such information elements were derived by the analysis toolkit module 260 (shown in figure 2). The graphical user interface module displays the selected subset of additional viewing windows simultaneously with the first viewing window 202 and, this example, second viewing window 204.

Advantageously, the above described graphical user interface allows a user to view simultaneously multiple information elements related to the labor progression. Another advantage of the present invention is that it allows a user to select which information elements to view for a given obstetrics patient. This provides the user with flexibility regarding what is being observed such that information elements considered to be more important by the user may be displayed and those considered of lesser importance may be fully or partially concealed on the display. This flexibility allows the clinical staff to assess more easily how labour is progressing by limiting the amount information that they are observing and need to process.

In the specific implementation depicted in figure 3a, the second tracing in the second viewing window 204 conveys a running average of a uterine contraction count associated with the obstetrics patient. The second viewing window 204 also includes information pertaining to a safety limit associated with the average of a uterine contraction count. The safety limit includes a threshold uterine contraction count illustrated a third tracing 290. In the example depicted, the threshold value is depicted by a dotted line positioned along a certain contraction count. The threshold value 290 marks a boundary between uterine contraction counts considered to be normal and contraction count considered as being associated to riskier situations. Optionally, the graphical user interface module is adapted for displaying a visual indicator in association with the second viewing window 204 when the running average of the uterine contraction count associated with the obstetrics patient exceeds the threshold uterine contraction count 290. Any suitable type of visual indicator may be used to draw attention to the additional viewing window 204. Examples of visual indicators that may be used include, without being limited to:
➢ Variations in color. For example, a color scheme may be established whereby certain colors are associated with varying levels of risk. Portions of the window may turn a certain color associated with a high level of risk when the fetal heart rate feature measurement exceeds an associated threshold value. A non-limiting example of a color scheme is green = normal; yellow: intermediate risk level; red: high level of risk however any suitable color scheme may be used;
➢ Variation in display intensity of the viewing window. For example, flashing or blinking of the viewing window may be used as a visual indicator to draw attention;
➢ Variation in the size of the size or position of the viewing window. For example, the appearance of the viewing window may be made to appear more prominently on the user interface or at a location that is more likely to draw the attention of the clinical staff;
➢ Causing the viewing window to be displayed or expanded. For example, in the case where the viewing window is not displayed on the user interface or is in collapsed form, automatically causing the viewing window to be displayed or expanded;
➢ Displaying a message prompting/alerting the clinical staff to observe what is going on in the additional viewing window 204.

Although the above described example of visual indicators have been described in the context of drawing attention to the additional viewing window 204, similar types of visual indicators may be used for drawing attention other viewing windows in a set of addition viewing windows. In an alternative embodiment, an audio signal, alone or in combination with the visual indicator, may be used to draw attention to an additional viewing window displaying a feature that has exceeded its associated safety limit.

In the example depicted in figure 3a, the graphical user interface module is depicted as including a set of controls 206m-o each of which, when actuated, displays or conceals an associated additional viewing window. Each control 206m-o is associated to respective feature measurements related to labour progression. In the example shown, the additional viewing windows associated to controls 206m, 206n, 206o are displayed simultaneously with the first viewing window 202 and second viewing window 204. In a specific example of implementation, whether the additional viewing windows associated to controls 206m, 206n, 206o convey information related to labor progression over time, the viewing windows are time aligned such as to allows the user to view the various information over the same time period.

In the example depicted in figure 3a, an additional viewing window is displayed conveying information related to the expected dilation of the cervix over time. This additional viewing window is associated with control 206m. More specifically, the additional viewing window displays over time the expected dilation of the cervix. This information is derived by the corresponding tool in the analysis toolkit module 260.

Although the controls 206m-o depicted in figure 3a are shown in association with a set of additional viewing windows that can be displayed or concealed, it will be appreciated that the control may be of any suitable form for allowing the user to select a subset of a set of additional viewing windows. For example, the control may be comprised of text boxes, drop down menus, handles, images (eg. icons) or any other suitable means on the user interface. The user may provide his selection through the user input device 118 which may be any one or a combination of the following: keyboard, pointing device, touch sensitive surface or speech recognition unit.

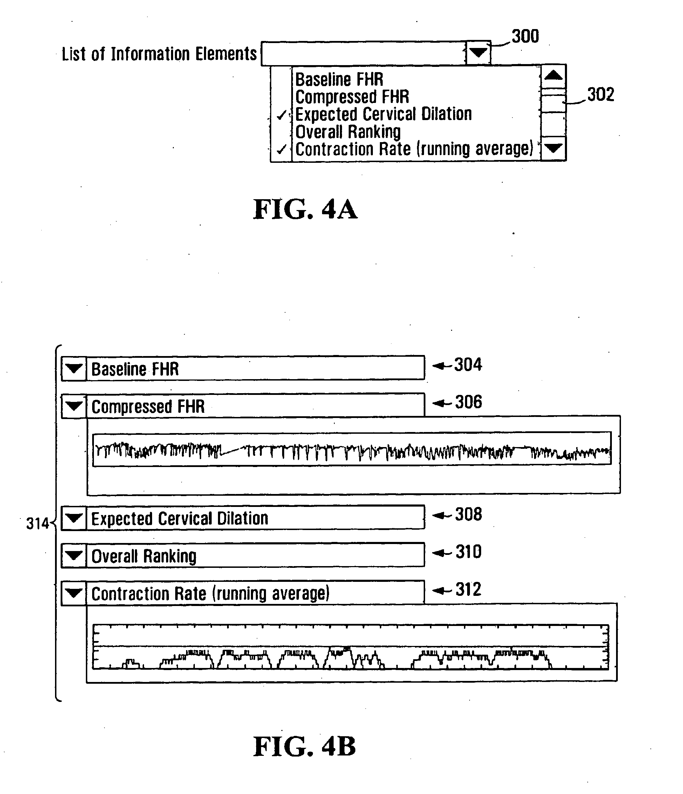

Specific examples of the type of controls that may be used in practical implementations will now be described with reference to figures 4a and 4b.

In Figure 4a, the control is in the form of a pull-down menu having an actuator button 300 that, when actuated, is operative for displaying or concealing a list of available additional information elements. The selected information elements are shown with a check mark (√) next to the information elements. The unselected information elements are shown without a check mark (√) next to the information elements. The pull-down menu further includes a sliding bar 302 for allowing additional information elements to be listed.

In Figure 4b, an alternative example of a set of controls 314 including controls 304 306 308 310 and 312 is depicted. Each control in the set of controls 314 is associated to a respective information element and allows a user to select the associated viewing window to be displayed or concealed. In the example depicted, the controls 304 306 308 310 and 312 are pull-down windows positioned one over the other with the time axis, when applicable, of each of the windows are aligned. The actuation of a given control causes the associated viewing window to be displayed (when the window had previously been concealed) or concealed (when the window had previously been displayed). It will be appreciated that the unselected windows may either be fully concealed (i.e. not displayed in any way) on the user interface or alternatively may be partially concealed.

In yet another embodiment, not shown in the figures, the set of possible viewing windows includes one or more groupings of viewing windows including at least two viewing windows. The user interface includes a control allowing the user to select a grouping viewing windows such as to allow the user to select two (2) or more viewing windows simultaneously. Preferably, the groupings of viewing windows are configured such that a given grouping includes viewing windows pertaining to related feature measurements or to feature measurements that should be considered as a group in order to provide useful to the clinical staff. For example, a grouping for a mother in pre-term labour could be provided, the grouping including viewing windows for displaying the contraction count, the tocolytic medication level and the fetal heart rate. The grouping for a mother in pre-term labour may omit cervical dilation and station. Alternatively, a grouping for a mother in term labour could be provided, the display including a grouping having viewing windows for displaying station and dilation. The grouping for a mother in term labour would likely omit tocolytic medication levels.

It is to be appreciated that the examples described are non-exhaustive and that such examples are provided for the purpose of illustration only. Suitable types of controls other than the ones described above may be used.

### Second Example

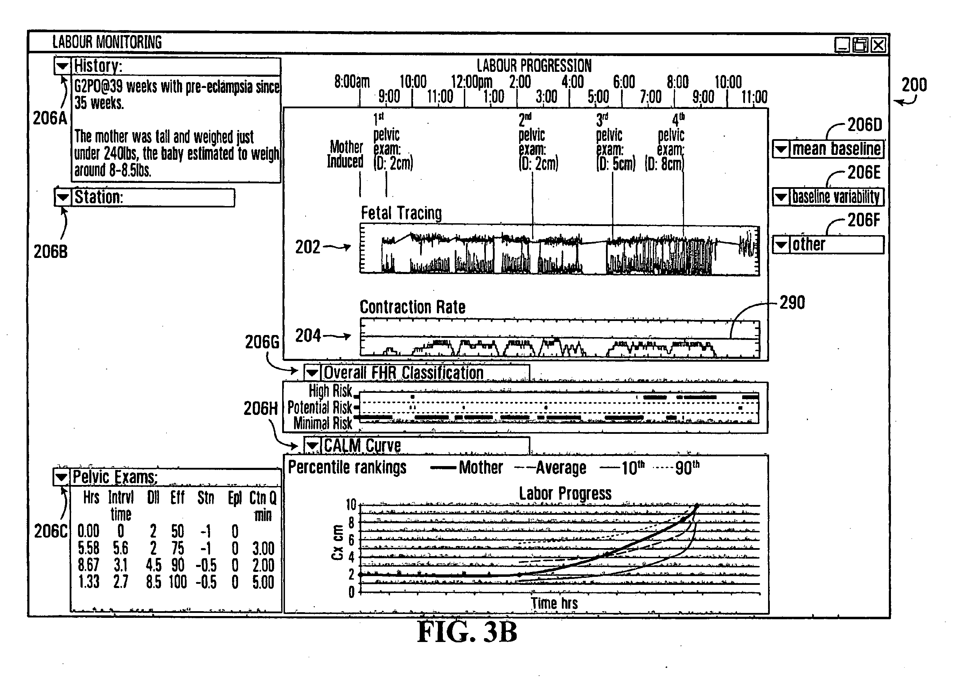

With reference to figure 3b, a second specific example of a graphical user interface module 200 implemented by graphical user interface module generator 262 (shown in figure 2) is shown. The graphical user interface module 200, similar to that depicted in figure 3a, includes a first viewing window 202 and a second viewing window 204. In the first viewing window 202, a first tracing indicative of a fetal heart rate signal is displayed and in the second viewing window 204, a second tracing indicative of a uterine contraction pattern associated with the obstetrics patient is displayed. The graphical user interface module also includes a set of controls 206a-h for allowing a user to select for display additional viewing windows from a set of possible viewing windows. As described above, the additional viewing windows in the set of additional viewing windows are adapted for conveying various information related to the assessment of labour progression for the obstetrics patient. Such information was derived by the analysis toolkit module 260 (shown in figure 2). The graphical user interface module displays the selected subset of additional viewing windows simultaneously with the first viewing window 202 and second viewing window 204.

In the specific implementation depicted in figure 3b, the second tracing in the second viewing window 204 conveys a running average of a uterine contraction count associated with the obstetrics patient. The second viewing window 204 also includes a third tracing 290 indicative of a threshold uterine contraction count.

In the example depicted in figure 3b, the graphical user interface module is depicted as including a set of controls 206a-h each of which, when actuated, displays or conceals an associated additional viewing window. Each control 206a-h is associated to respective information elements. In the example shown, the additional viewing windows associated to controls 206a, 206b, 206c, 206g and 206h are displayed simultaneously with the first viewing window 202 and second viewing window 204. The additional viewing windows corresponding to un-selected information elements and associated to controls 206d, 206e and 206f are not displayed on the graphical user interface.

In the example depicted in figure 3b, an additional viewing window is displayed conveying information related to levels of risk associated with labour. This additional viewing window is associated with control 206g. More specifically, the additional viewing window displays over a time segment data conveying levels of risk associated with labour. In the example shown in figure 3b, the time segment is divided into a plurality of sub-segments and data conveying a respective level of risk associated with labour for an obstetrics patient is depicted for each sub-segment.

In the example depicted in figure 3b, an additional viewing window is displayed conveying information related to the expected dilation of the cervix over time. This additional viewing window is associated with control 206h. More specifically, the additional viewing window displays over a time the expected dilation of the cervix. This information is derived by the corresponding tool in the analysis toolkit module 260.

Although the controls 206a-h depicted in figure 3b are shown in association with a set of additional viewing windows that can be displayed or concealed, it will be appreciated that the control may be of any suitable form for allowing the user to select a subset of a set of additional viewing windows. For example, the control may be comprised of text boxes, drop down menus, handles, images (eg. icons) or any other suitable means on the user interface. The user may provide his selection through the user input device 118 which may be any one or a combination of the following: keyboard, pointing device, touch sensitive surface or speech recognition unit.

In a variant, the graphical user interface module displays one or more additional viewing windows on the basis of a user profile. In a specific implementation, graphical user interface module makes use of a user identifier obtained, for example, during a log-in process, to determine a subset of viewing windows to be displayed for a given user. The set of viewing windows to be displayed for a given user may be specified in a "user preferences" database whereby user identifiers are mapped to a set of identifiers corresponding to respective subsets of viewing windows. Advantageously, this allows users to have a preferred subset of viewing windows that are displayed when using the interface. Suitable functionality for allowing the user to configure and or modify the entries in the user preferences database may be provided. Manners in which to implement such functionality is well-known the art of software design and the specific manner in which the configuration and modification functionality is provided is not critical to the invention and as such will not be described further here.

In yet another variant, the graphical user interface module is adapted for enabling a user to select a profile from a group of possible profiles, at least some profiles being associated to two (2) or more viewing windows. Suitable functionality for allowing the user to configure and or modify the profiles in the group of possible profiles may also be provided. Manners in which to implement such functionality is well-known the art of software design and the specific manner in which the configuration and modification functionality is provided is not critical to the invention and as such will not be described further here.

### Third Example

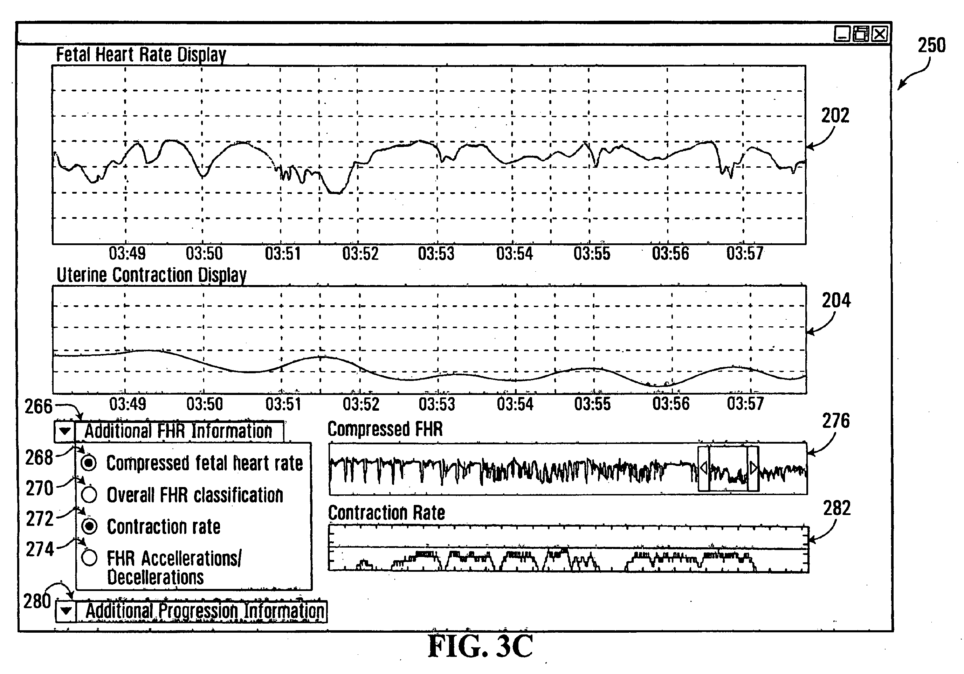

With reference to figure 3c, a second specific example of a graphical user interface module 250 implemented by graphical user interface module generator 262 (shown in figure 2) is shown. As in the case of the graphical user interface module 200 of figures 3a and 3b, the graphical user interface module 250 includes a first viewing window 202 and a second viewing window 204. In the first viewing window 202, a first tracing indicative of a fetal heart rate signal is displayed and in the second viewing window 204, a second tracing indicative of a uterine contraction pattern associated with the obstetrics patient is displayed. The graphical user interface module also includes a control 266 for allowing a user to select additional viewing windows for display from a set of possible viewing windows. As described above, the additional viewing windows in the set of possible viewing windows are adapted for conveying various information elements related to the assessment of labour progression for the obstetrics patient. Such information elements were derived by the analysis toolkit module 260 (shown in figure 2). The user may provide his selection through the user input device 118 which may be any one or a combination of the following: keyboard, pointing device, touch sensitive surface or speech recognition unit. The graphical user interface module displays the selected subset of additional viewing windows simultaneously with the first viewing window 202 and second viewing window 204.

In figure 3c, the control 266 is in the form of a pull-down menu. The pull down menu includes a plurality of selection buttons 268 270 272 274 associated to respective information elements, each of the selection buttons 268 270 272 274 being adapted to be actuated or de-actuated to either select or un-select the associated information element. In the example shown, selection buttons 268 and 272 are actuated to select respective associated information elements "Compressed fetal heart rate" and "Contraction rate". As shown, additional viewing windows 276 282 corresponding to selected information elements "Compressed fetal heart rate" and "Contraction rate" are displayed simultaneously with the first viewing window 202 and second viewing window 204. The additional viewing windows corresponding to un-selected information elements, namely "Overall FHR classification" and "FHR accelerations/decelerations" are not displayed on the graphical user interface.

In a specific example of implementation, the additional viewing windows are combined into several sets of additional viewing windows. The combination may be effected on the basis of any suitable logical/heuristic rules. In a specific example of implementation, of the type depicted in figure 3c, the additional viewing windows are combined into a first set conveying information data elements derived from a fetal heart rate signal and a second set conveying information data elements derived at least in part on the basis of clinical measurements obtained from the obstetrics patient. Optionally, each set of additional viewing windows is associated with a respective control, namely controls 266 and 280. Control 280 is displayed on the graphical user interface for allowing a user to select a subset of the second set of additional viewing windows. The selected subset of the second set of additional viewing windows is then displayed simultaneously with the first viewing window and second viewing window.

### Additional Viewing Windows

As indicated above, the graphical user interface module includes a control for allowing a user to select additional viewing windows from a set of additional viewing windows. The viewing windows in the set of possible viewing windows are adapted for conveying various additional information elements related to the assessment of labour progression for the obstetrics patient. The specific type of information elements to be displayed by the graphical user interface module in the possible viewing windows will vary from one implementation to the other and will depend on the functionality implemented by the toolkit module 260 (shown in figure 2).

Certain additional viewing window in the set of possible viewing windows are adapted to convey information elements derived at least in part on the basis of the fetal heart rate signal. Such information elements are typically derived by processing the fetal heart rate and may include, without being limited to:
1) fetal heart rate feature measurements such as:
   - mean baseline;
   - mean baseline variability;
   - decelerations;
   - accelerations;
2) fetal heart rate classification;
3) others...

In a specific example of implementation, the fetal heart rate feature measurements may be represented as tracings of the fetal heart rate feature measurements over time or in numerical format (text format) indicating the values of the feature measurements over time.

In accordance with a specific implementation, in addition to the fetal heart rate measurements, information element indicating a safety limit for a feature measurement related to labor progression is also displayed. The information element indicating a safety limit for a feature measurement may include, for example, a threshold value associated to the fetal heart rate feature measurement. In a specific example, each the information element includes one or more threshold values associated to the corresponding fetal heart rate feature measurement. The one or more threshold values may be depicted graphically as tracings or in numerical format (text format).

In a specific example of implementation, threshold values are depicted by dotted lines positioned on the graphs at levels corresponding to the threshold values for the various feature measurements. The threshold values mark a boundary between feature measurements (or rankings) considered having a lower level of risk and feature measurements (or rankings) considered having a higher level of risk. It will be readily apparent that multiple thresholds may be used for marking boundaries between different levels of risk (eg. low, intermediate, high etc...). The number of threshold levels for a given feature measurement will typically depend on the type of feature element measurement. The manner in which the threshold levels are selected and well as the number of threshold levels is not critical to the invention and as such will not be described further here.

In a specific example of implementation, the graphical user interface module is adapted for displaying a visual indicator in association with a viewing window in the set of additional viewing windows, the visual indicator conveying that a fetal heart rate feature measurement has exceeded an associated safety limit. For example, the visual indicator may be used to draw the attention of the clinical staff to a feature measurement that has exceeded a threshold and that is now consider to be associated to a high level of risk. For example, in cases where the additional viewing window associated with the fetal heart rate feature measurement that has exceeded its associated threshold value is fully or partially displayed, the visual indicator may be displayed in conjunction with that additional viewing window. Any suitable type of visual indicator may be used to draw attention to the additional viewing window. Examples of visual indicators that may be used include, without being limited to:
➢ Variations in color. For example, a color scheme may be established whereby certain colors are associated with varying levels of risk. Portions of the window may turn a certain color associated with a high level of risk when the fetal heart rate feature measurement exceeds an associated threshold value. A non-limiting example of a color scheme is green = normal; yellow: intermediate risk level; red: high level of risk however any suitable color scheme may be used;
➢ Variation in display intensity of the additional viewing window. For example, flashing or blinking of the additional viewing window may be used as a visual indicator to draw attention;
➢ Variation in the size of the size or position of the additional viewing window. For example, the appearance of the additional viewing window may be made to appear more prominently on the user interface or at a location that is more likely to draw the attention of the clinical staff.
➢ Displaying a message prompting/alerting the clinical staff to observe what is going on in the additional viewing window.

Optionally, in cases where the additional viewing window associated with the fetal heart rate feature measurement that has exceeded its associated threshold value is not at least partially displayed, the graphical user interface module is adapted to cause the additional viewing window to be displayed without the intervention of the user. A visual indicator may then (optionally) also be displayed in conjunction with that additional viewing window.

It will readily be appreciated that the list of functionality and additional viewing windows is non-exhaustive and has been provided for the purpose of illustration only.

### Specific Physical Implementation

Those skilled in the art should appreciate that in some embodiments of the invention, all or part of the functionality previously described herein with respect to the apparatus implementing a user interface for displaying labour related information may be implemented as pre-programmed hardware or firmware elements (e.g., application specific integrated circuits (ASICs), electrically erasable programmable read-only memories (EEPROMs), etc.), or other related components.

In other embodiments of the invention, all or part of the functionality previously described herein with respect to the apparatus for implementing a graphical user interface module for displaying labour related information may be implemented as software consisting of a series of instructions for execution by a computing unit. The series of instructions could be stored on a medium which is fixed, tangible and readable directly by the computing unit, (e.g., removable diskette, CD-ROM, ROM, PROM, EPROM or fixed disk), or the instructions could be stored remotely but transmittable to the computing unit via a modem or other interface device (e.g., a communications adapter) connected to a network over a transmission medium. The transmission medium may be either a tangible medium (e.g., optical or analog communications lines) or a medium implemented using wireless techniques (e.g., microwave, infrared or other transmission schemes).

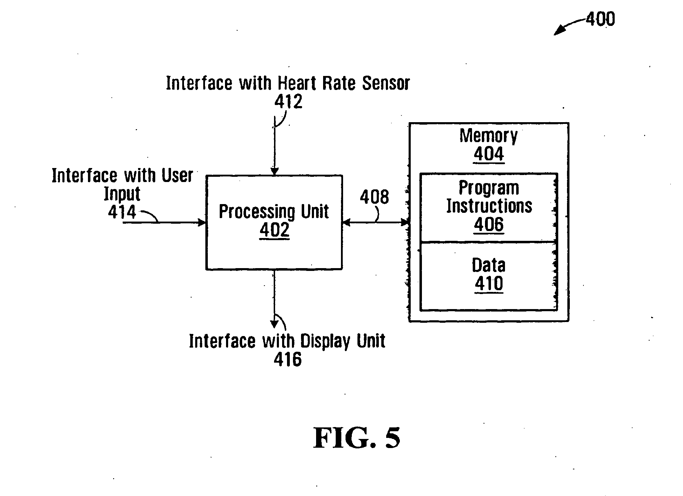

The apparatus implementing a user interface for displaying labour related information may be configured as a computing unit of the type depicted in figure 5, including a processing unit 402 and a memory 404 connected by a communication bus 408. The memory 404 includes data 410 and program instructions 406. The processing unit 402 is adapted to process the data 410 and the program instructions 406 in order to implement the functional blocks described in the specification and depicted in the drawings. In a non-limiting implementation, the program instructions 406 implement the functionality of processing unit 106 described above. The computing unit 400 may also comprise a number of interfaces 412 414 416 for receiving or sending data elements to external devices. For example, interface 412 is used for receiving data streams indicative of a fetal heart rate signal and interface 414 is used for receiving a control signal from the user indicating the subset of labour related information data to be displayed. Interface 416 is for releasing a signal causing a display unit to display the user interface generated by the program instructions 406.

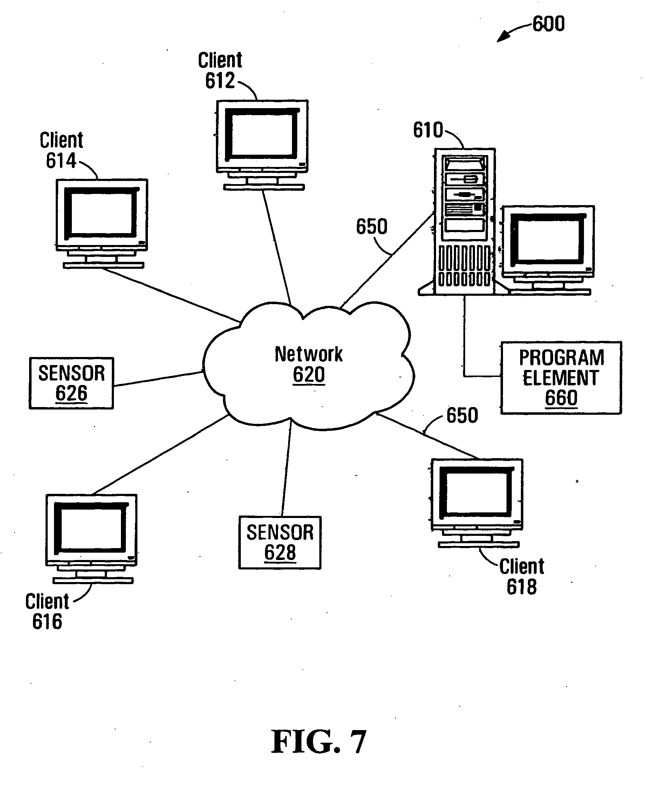

It will be appreciated that the system for implementing a user interface for displaying labour related information may also be of a distributed nature where the data is collected at one location and transmitted over a network to a server unit implementing the graphical user interface. The server unit may then transmit a signal for causing a display unit to display the graphical user interface. The display unit may be located in the same location as the data was collected, in the same location as the server unit or in yet another location. Figure 7 illustrates a network-based client-server system 600 for displaying heart rate information. The client-server system 600 includes a plurality of client systems 612 614 616 618 connected to a server system 610 through network 620. The communication links 650 between the client systems 612 614 616 618 and the server system 610 can be metallic conductors, optical fibers or wireless. The network 620 may be any suitable network including but not limited to a global public network such as the Intranet, a private network and a wireless network. The server 610 may be adapted to process and issue signals to display multiple heart rate signals originating from multiple sensors 626 628 concurrently using suitable methods known in the computer related arts.

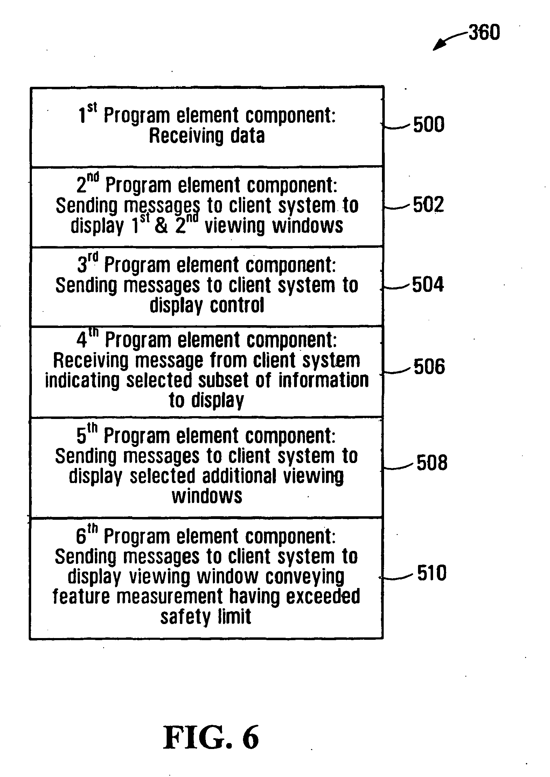

The server system 610 includes a program element 660 for execution by a CPU. Program element 660 implements similar functionality as program instructions 406 (shown in figure 5) and includes the necessary networking functionality to allow the server system 610 to communicate with the client systems 612 614 616 618 over network 620. In a non-limiting implementation, program element 660 includes a number of program element components, each program element components implementing a respective portion of the functionality of the user interface for displaying heart rate information. Figure 6 shows a non-limiting example of the architecture of program element 660 at the server system. As shown, the program element 660 includes six program element components:
1. the first program element component 500 is executed on server system 610 and is for receiving signals conveying labour information;
2. the second program element component 502 is executed on server system 610 and is for sending messages to a client system, say client system 614, for causing client system 614 to displaying a first viewing window selected from a set of possible viewing windows, each viewing window in the set of possible viewing windows conveying a feature measurement related to labour progression, at least one viewing window in the set of possible viewing windows conveying:
   i) a given feature measurement; and
   ii) a safety limit associated to the given feature measurement;
3. the third program element component 504 is executed on server system 610 and is for sending messages to client system 614 for causing client system 614 to display a control allowing a user to select a subset of the set of possible viewing windows;
4. the fourth program element component 506 is executed on server system 610 and is for receiving a message from client system 614 indicative of a selected subset of viewing windows;
5. the fifth program element component 508 is executed on server system 610 and is for sending messages to client system 614 for causing client system 614 to display, simultaneously with the first viewing window, the selected subset of additional viewing windows;
6. the fifth program element component 508 is executed on server system 610 and is for sending messages to client system 614 for causing client system 614 to display, simultaneously with the first viewing window, a viewing window conveying a given feature measurement in response to the given feature measurement exceeding the associated safety limit associated to the given feature measurement.

Those skilled in the art should further appreciate that the program instructions may be written in a number of programming languages for use with many computer architectures or operating systems. For example, some embodiments may be implemented in a procedural programming language (e.g., "C") or an object oriented programming language (e.g., "C++" or "JAVA").

Although the present invention has been described in considerable detail with reference to certain preferred embodiments thereof, variations and refinements are possible. Therefore, the scope of the invention should be limited only by the appended claims and their equivalents.

## Claims

1. A method for displaying labour related information, said method comprising:
a) receiving signals conveying labour progression information associated with an obstetrics patient;
b) processing the signals at least in part to derive measurements over time of a plurality of features related to labour progression, a given feature in the plurality of features being associated with a corresponding safety limit;
c) processing measurements of the given feature to determine if they exceed the corresponding safety limit;
d) displaying on a display module a first viewing window conveying measurements over time of a first feature related to labour progression of the obstetric patient;
said method being **characterized in that** it comprises:
e) displaying on the display module at least one control allowing a user to provide an input to select an additional viewing window from a set of possible additional viewing windows, the set of possible additional viewing windows including at least some windows associated with features related to labour progression of the obstetric patient, one viewing window in the set of possible additional viewing windows conveying:
(i) measurements associated with the given feature; and
(ii) the corresponding safety limit associated with the given feature;
f) receiving selection information conveying the additional viewing window selected by the user using the at least one control and using said selection information to cause the display module to display the selected additional viewing window simultaneously with the first viewing window so that labour related information being displayed on the display module is customized at least in part based on the input provided by the user, wherein the selected additional viewing window:
(i) conveys measurements over time of a second feature related to labour progression of the obstetric patient; and
(ii) is displayed in a substantially time aligned manner with the first viewing window to allow the user to view measurements of the first feature and measurements of the second feature over concurrent time periods;
g) in response to measurements of the given feature exceeding the corresponding safety limit, displaying on the display module information to attract the attention of the user to the one viewing window in the set of possible viewing windows conveying measurements of the given feature.

2. A method as defined in claim 1, wherein in response to measurements of the given feature exceeding the corresponding safety limit, the one viewing window in the set of possible viewing windows conveying measurements of the given feature is displayed in a substantially time aligned manner with the first viewing window and the selected additional viewing window to allow the user to view measurements of the first feature, measurements of the second feature and measurements of the given feature over concurrent time periods.

3. A method as defined in claim 1, wherein in response to measurements of the given feature exceeding the corresponding safety limit, the certain viewing window conveying the given feature measurement is displayed concurrently with the first viewing window and the selected additional viewing window.

4. A method as defined in any one of the preceding claims, wherein in response to measurements of the given feature exceeding the corresponding safety limit, a visual indicator is displayed in association with the certain viewing window conveying the given feature measurement to attract the attention of the user to the viewing window conveying the given feature measurement.

5. A method as defined in any one of the preceding claims, said method comprising:
a) receiving a signal conveying a user identifier associated with the user of the system;
b) selecting a viewing window from the set of possible viewing windows at least in part based on the signal conveying the user identifier;
c) displaying the viewing window selected at least in part based on the signal conveying the user identifier concurrently with the first viewing window and the selected additional viewing window.

6. A method as defined in any one of the preceding claims, wherein said set of possible viewing windows includes a viewing window conveying over a first time segment levels of risk associated with labour progression.

7. A method as defined in claim 6, wherein for each time sub-segment of said first time segment, said method comprises processing the signals conveying labour progression information to derive data conveying respective levels of risk associated with labour progression.

8. A method as defined in any one of the preceding claims, wherein at least one viewing window in said set of possible viewing windows conveys information elements derived at least in part based on clinical measurements obtained from the obstetrics patient.

9. A method as defined in claim 8, wherein said set of possible viewing windows includes a viewing window displaying data conveying expected cervical dilations over time.

10. A method as defined in any one of the preceding claims, wherein at least one possible viewing window in said set of possible viewing windows conveys measurements of a fetal heart rate feature over time.

11. A method as defined in any one of the preceding claims, wherein a viewing window in the set of possible viewing windows conveys a tracing indicative of a uterine contraction pattern.

12. A method as defined in claim 11, wherein the uterine contraction pattern is a running average of a uterine contraction count associated with the obstetrics patient.

13. A method as defined in any one of the preceding claims, wherein said at least one control includes a selection box.

14. A method as defined in any one of claims 1 to 13, wherein said at least one control is one of a set of controls, said method comprises displaying the set of controls, each control in said set of controls being associated to a respective viewing window in the set of possible viewing windows, each control allowing a user to select an associated viewing window.

15. A method as defined in any one of the preceding claims, wherein said at least one control allows the user to select the subset of viewing windows from the set of possible viewing windows by using an input device selected from the set consisting of a mouse, keyboard, pointing device, speech recognition unit and touch sensitive screen.

16. A computer readable storage medium storing a program element suitable for execution by a CPU, said program element implementing the method for displaying labour related information described in any one of claims 1 to 15.

17. A server system storing a program element for execution by a CPU, said program element implementing the method for displaying labour related information described in any one of claims 1 to 15.

18. An apparatus implementing a user interface for displaying labour related information, said apparatus comprising input means for receiving signals conveying labour information and processing means in communication with said input means, said processing means being adapted for implementing the method described in any one of claims 1 to 15.

19. A labour monitoring system comprising:
a) a first sensor for receiving a signal indicative of a fetal heart rate;
b) a second sensor for receiving a uterine contraction signal associated with an obstetrics patient;
c) an apparatus implementing a graphical user interface module for displaying labour related information, said apparatus comprising:
i) first input means in communication with said first sensor for receiving the fetal heart rate signal;
ii) second means in communication with said second sensor for receiving the uterine contraction signal;
iii) processing means in communication with said first and second input means;
d) display means in communication with said apparatus;
e) **characterized in that** the processing means of said apparatus are adapted for implementing the method described in any one of claims 1 to 15.

20. A client-server system implementing a graphical user interface module for displaying labour related information, said client-server system comprising a client system and a server system, said client system and said server system operative to exchange messages over a data network, said server system storing a program element for execution by a CPU, **characterized in that** said program element when executing on said CPU implements the method described in any one of claims 1 to 15.

21. A client-server system as defined in claim 20, wherein the data network is the Internet.

## Patentansprüche

1. Verfahren zum Anzeigen von wehenbezogenen Informationen, welches Verfahren aufweist:
a) Empfangen von Signalen, die mit einer Geburtshilfepatientin assoziierte Wehenprogressionsinformationen übermitteln;
b) zumindest teilweises Verarbeiten der Signale, um zeitabhängige Messungen mehrerer auf die Wehenprogression bezogener Merkmale abzuleiten, wobei ein gegebenes Merkmal der mehreren Merkmale mit einer entsprechenden Sicherheitsgrenze assoziiert ist;
c) Verarbeiten von Messungen des gegebenen Merkmals, um zu bestimmen, ob sie die entsprechende Sicherheitsgrenze überschreiten;
d) Anzeigen eines ersten Betrachtungsfensters, das zeitabhängige Messungen eines ersten Merkmals, das auf eine Wehenprogression der Geburtshilfepatientin bezogen ist, übermittelt, auf einem Anzeigemodul;
welches Verfahren **dadurch gekennzeichnet ist, dass** es aufweist:
e) Anzeigen zumindest einer Steuerung, die einem Benutzer ermöglicht, eine Eingabe zur Auswahl eines zusätzlichen Betrachtungsfensters aus einem Satz von zusätzlichen möglichen Betrachtungsfenstern vorzusehen, auf dem Anzeigemodul, wobei der Satz von zusätzlichen möglichen Betrachtungsfenstern zumindest einige Fenster enthält, die mit Merkmalen assoziiert sind, die auf eine Wehenprogression der Geburtshilfepatientin bezogen sind, wobei ein Betrachtungsfenster in dem Satz von zusätzlichen möglichen Betrachtungsfenstern übermittelt:
(i) mit dem gegebenen Merkmal assoziierte Messungen; und
(ii) die entsprechende Sicherheitsgrenze, die mit dem gegebenen Merkmal assoziiert ist;
f) Empfangen von Auswahlinformationen, die das zusätzliche Betrachtungsfenster, das von dem Benutzer unter Verwendung der zumindest einen Steuerung ausgewählt wurde, übermitteln, und Verwenden der Auswahlinformationen, um zu bewirken, dass das Anzeigemodul das ausgewählte zusätzliche Betrachtungsfenster gleichzeitig mit dem ersten Betrachtungsfenster so anzeigt, dass wehenbezogene Informationen, die auf dem Anzeigemodul angezeigt werden, zumindest teilweise auf der Grundlage der von dem Benutzer vorgesehenen Eingabe angepasst werden, wobei das ausgewählte zusätzliche Betrachtungsfenster:
(i) zeitabhängige Messungen eines zweiten Merkmals, das auf die Wehenprogression der Geburtshilfepatientin bezogen ist, übermittelt; und
(ii) in einer im Wesentlichen zeitausgerichteten Weise mit dem ersten Betrachtungsfenster angezeigt wird, um dem Benutzer zu ermöglichen, Messungen des ersten Merkmals und Messungen des zweiten Merkmals über gleichzeitige Zeitperioden zu betrachten;
g) als Antwort auf Messungen des gegebenen Merkmals, die die entsprechende Sicherheitsgrenze überschreiten, Anzeigen von Informationen auf dem Anzeigemodul, um die Aufmerksamkeit des Benutzers auf das eine Betrachtungsfenster in dem Satz von möglichen Betrachtungsfenstern, das Messungen des gegebenen Merkmals übermittelt, zu lenken.

2. Verfahren nach Anspruch 1, bei dem als Antwort auf Messungen des gegebenen Merkmals, das die entsprechende Sicherheitsgrenze überschreitet, das eine Betrachtungsfenster in dem Satz von möglichen Betrachtungsfenstern, das Messungen des gegebenen Merkmals übermittelt, in einer im Wesentlichen zeitausgerichteten Weise mit dem ersten Betrachtungsfenster und dem ausgewählten zusätzlichen Betrachtungsfenster angezeigt wird, um dem Benutzer zu ermöglichen, Messungen des ersten Merkmals, Messungen des zweiten Merkmals und Messungen des gegebenen Merkmals während gleichzeitiger Zeitperioden zu betrachten.

3. Verfahren nach Anspruch 1, bei dem als Antwort auf Messungen des gegebenen Merkmals, das die entsprechende Sicherheitsgrenze überschreitet, das gewisse Betrachtungsfenster, das die gegebene Merkmalsmessung übermittelt, gleichzeitig mit dem ersten Betrachtungsfenster und dem ausgewählten zusätzlichen Betrachtungsfenster angezeigt wird.

4. Verfahren nach einem der vorhergehenden Ansprüche, bei dem als Antwort auf Messungen des gegebenen Merkmals, das die entsprechende Sicherheitsgrenze überschreitet, ein visueller Indikator in Verbindung mit dem gewissen Betrachtungsfenster, das die Messung des gegebenen Merkmals übermittelt, angezeigt wird, um die Aufmerksamkeit des Benutzers auf das Betrachtungsfenster zu lenken, das die Messung des gegebenen Merkmals übermittelt.

5. Verfahren nach einem der vorhergehenden Ansprüche, welches Verfahren aufweist:
a) Empfangen eines Signals, das einen Benutzerkennzeichner, der mit dem Benutzer des Systems assoziiert ist, übermittelt;
b) Auswählen eines Betrachtungsfensters aus dem Satz von möglichen Betrachtungsfenstern zumindest teilweise auf der Grundlage des den Benutzerkennzeichner übermittelnden Signals;
c) Anzeigen des Betrachtungsfensters, das zumindest teilweise auf der Grundlage des den Benutzerkennzeichner übermittelnden Signals ausgewählt wurde, gleichzeitig mit dem ersten Betrachtungsfenster und dem ausgewählten zusätzlichen Betrachtungsfenster.

6. Verfahren nach einem der vorhergehenden Ansprüche, bei dem der Satz von möglichen Betrachtungsfenstern ein Betrachtungsfenster enthält, das während eines ersten Zeitsegments mit der Wehenprogression assoziierte Gefahrenpegel übermittelt.

7. Verfahren nach Anspruch 6, bei dem für jedes Zeitsubsegment des ersten Zeitsegments das Verfahren eine Verarbeitung der Signale, die Wehenprogressionsinformationen übermitteln, aufweist, um Daten abzuleiten, die jeweilige mit der Wehenprogression assoziierte Gefahrenpegel übermitteln.

8. Verfahren nach einem der vorhergehenden Ansprüche, bei dem zumindest ein Betrachtungsfenster in dem Satz von möglichen Betrachtungsfenstern Informationselemente übermittelt, die zumindest teilweise auf der Grundlage von klinischen Messungen, die von der Geburtshilfepatientin erhalten wurden, abgeleitet sind.

9. Verfahren nach Anspruch 8, bei dem der Satz von möglichen Betrachtungsfenstern ein Betrachtungsfenster enthält, das Daten anzeigt, die erwartete Gebärmutterhalsdehnungen über die Zeit übermitteln.

10. Verfahren nach einem der vorhergehenden Ansprüche, bei dem zumindest ein mögliches Betrachtungsfenster in dem Satz von möglichen Betrachtungsfenstern Messergebnisse eines fetalen Herzfrequenzmerkmals über die Zeit übermittelt.

11. Verfahren nach einem der vorhergehenden Ansprüche, bei dem ein Betrachtungsfenster in dem Satz von möglichen Betrachtungsfenstern eine Aufzeichnung übermittelt, die ein Gebärmutter-Kontraktionsmuster anzeigt.

12. Verfahren nach Anspruch 11, bei dem das Gebärmutter-Kontraktionsmuster ein laufender Durchschnitt einer Gebärmutter-Kontraktionszählung, die mit der Geburtshilfepatientin assoziiert ist , ist.

13. Verfahren nach einem der vorhergehenden Ansprüche, bei dem die zumindest eine Steuerung ein Auswahlfeld enthält.

14. Verfahren nach einem der Ansprüche 1 bis 13, bei dem die zumindest eine Steuerung eine aus einem Satz von Steuerungen ist, wobei das Verfahren die Anzeige des Satzes von Steuerungen aufweist und jede Steuerung in dem Satz von Steuerungen mit einem jeweiligen Betrachtungsfenster in dem Satz von möglichen Betrachtungsfenstern assoziiert ist, und jede Steuerung einem Benutzer ermöglicht, ein assoziiertes Betrachtungsfenster auszuwählen.

15. Verfahren nach einem der vorhergehenden Ansprüche, bei dem die zumindest eine Steuerung dem Benutzer ermöglicht, den Subsatz von Betrachtungsfenstern aus dem Satz von möglichen Betrachtungsfenstern auszuwählen durch Verwendung einer Eingabevorrichtung, die ausgewählt ist aus dem Satz bestehend aus einer Maus, einer Tastatur, einer Zeigevorrichtung, einer Spracherkennungseinheit und einem berührungsempfindlichen Bildschirm.

16. Computerlesbares Speichermedium, das ein Programmelement speichert, das geeignet für die Ausführung durch eine CPU ist, wobei das Programmelement das Verfahren zum Anzeigen von wehenbezogenen Informationen, das in jedem der Ansprüche 1 bis 15 beschrieben ist, realisiert.

17. Serversystem, das ein Programmelement für die Ausführung durch eine CPU speichert, wobei das Programmelement das Verfahren zum Anzeigen von wehenbezogenen Informationen, das in jedem der Ansprüche 1 bis 15 beschrieben ist, realisiert.

18. Vorrichtung, die eine Benutzerschnittstelle zum Anzeigen von wehenbezogenen Informationen realisiert, wobei die Vorrichtung Eingabemittel zum Empfangen von Signalen, die Weheninformationen übermitteln, und Verarbeitungsmittel in Kombination mit den Eingabemitteln aufweist, und wobei die Verarbeitungsmittel ausgebildet sind zum Realisieren des in jedem der Ansprüche 1 bis 15 beschriebenen Verfahrens.

19. Wehenüberwachungssystem, welches aufweist:
a) einen ersten Sensor zum Empfangen eines die fetale Herzfrequenz anzeigenden Signals;
b) einen zweiten Sensor zum Empfangen eines Gebärmutter-Kontraktionssignals, das mit einer Geburtshilfepatientin assoziiert ist;
c) eine Vorrichtung, die ein Benutzerschnittstellenmodul für Grafiken zum Anzeigen von wehenbezogenen Informationen realisiert, welche Vorrichtung aufweist:
(i) erste Eingabemittel in Kommunikation mit dem ersten Sensor zum Empfangen des fetalen Herzfrequenzsignals;
(ii) zweite Mittel in Kommunikation mit dem zweiten Sensor zum Empfangen des Gebärmutter-Kontraktionssignals;
(iii) Verarbeitungsmittel in Kommunikation mit den ersten und den zweiten Eingabemitteln;
d) Anzeigemittel in Kommunikation mit der Vorrichtung;
e) **dadurch gekennzeichnet, dass** die Verarbeitungsmittel der Vorrichtung ausgebildet sind zum Realisieren des in jedem der Ansprüche 1 bis 15 beschriebenen Verfahrens.

20. Client-Server-System, das ein Benutzerschnittstellenmodul für Grafiken zum Anzeigen von wehenbezogenen Informationen realisiert, welches Client-Server-System ein Clientsystem und ein Serversystem aufweist, wobei das Clientsystem und das Serversystem wirksam sind, um Botschaften über ein Datennetzwerk auszutauschen, und das Serversystem ein Programmelement zum Ausführen durch eine CPU speichert, **dadurch gekennzeichnet, dass** das Programmelement, wenn es von der CPU ausgeführt wird, das in jedem der Ansprüche 1 bis 15 beschriebene Verfahren realisiert.

21. Client-Server-System nach Anspruch 20, bei dem das Datennetzwerk das Internet ist.

## Revendications

1. Procédé d'affichage d'informations liées au travail, ledit procédé comprenant :
a) la réception de signaux fournissant des informations sur la progression du travail associées à une patiente en obstétrique ;
b) le traitement des signaux pour dériver au moins en partie les mesures au fil du temps d'une pluralité de caractéristiques liées à la progression du travail, une caractéristique donnée dans la pluralité de caractéristiques étant associée à une limite de sécurité correspondante ;
c) le traitement des mesures de la caractéristique donnée pour déterminer si elles dépassent la limite de sécurité correspondante ;
d) l'affichage sur un module d'affichage d'une première fenêtre de visualisation fournissant les mesures au fil du temps d'une première caractéristique liée à la progression du travail de la patiente en obstétrique ;
ledit procédé étant **caractérisé en ce qu'**il comprend :
e) l'affichage sur le module d'affichage d'au moins une commande permettant à un utilisateur de procéder à une entrée pour sélectionner une fenêtre de visualisation supplémentaire parmi un ensemble de fenêtres de visualisation possibles, l'ensemble de fenêtres de visualisation possibles comportant au moins certaines fenêtres associées aux caractéristiques liées à la progression du travail de la patiente en obstétrique, une fenêtre de visualisation de l'ensemble de fenêtres de visualisation supplémentaires possibles fournissant :
(i) des mesures associées à la caractéristique donnée ; et
(ii) la limite de sécurité correspondante associée à la caractéristique donnée ;
f) la réception d'informations de sélection fournissant la fenêtre de visualisation supplémentaire sélectionnée par l'utilisateur à l'aide de l'au moins une commande et l'utilisation desdites informations de sélection pour que le module d'affichage affiche la fenêtre de visualisation supplémentaire sélectionnée simultanément avec la première fenêtre de visualisation de sorte que les informations liées au travail affichées sur le module d'affichage sont personnalisées au moins en partie sur la base de l'entrée fournie par l'utilisateur, la fenêtre de visualisation supplémentaire sélectionnée :
(i) fournissant les mesures au fil du temps d'une seconde caractéristique liée à la progression du travail de la patiente en obstétrique ; et
(ii) étant affichée d'une manière sensiblement alignée dans le temps avec la première fenêtre de visualisation pour permettre à l'utilisateur de visualiser les mesures de la première caractéristique et les mesures de la seconde caractéristique sur des périodes de temps concurrentes ;
g) en réponse au dépassement par les mesures de la caractéristique donnée de la limite de sécurité correspondante, l'affichage sur le module d'affichage d'informations pour attirer l'attention de l'utilisateur sur la fenêtre de visualisation de l'ensemble de fenêtres de visualisation possibles fournissant les mesures de la caractéristique donnée.

2. Procédé selon la revendication 1, dans lequel en réponse au dépassement par les mesures de la caractéristique donnée de la limite de sécurité correspondante, la fenêtre de visualisation de l'ensemble de fenêtre de visualisation possibles fournissant les mesures de la caractéristique donnée est affichée d'une manière sensiblement alignée dans le temps avec la première fenêtre de visualisation et la fenêtre de visualisation supplémentaire sélectionnée pour permettre à l'utilisateur de visualiser les mesures de la première caractéristique, les mesures de la deuxième caractéristique et les mesures de la caractéristique donnée sur des périodes de temps concurrentes.

3. Procédé selon la revendication 1, dans lequel en réponse aux mesures du dépassement par la caractéristique donnée de la limite de sécurité correspondante, la certaine fenêtre de visualisation fournissant la mesure de la caractéristique donnée est affichée en parallèle avec la première fenêtre de visualisation et la fenêtre de visualisation supplémentaire sélectionnée.

4. Procédé selon l'une quelconque des revendications précédentes, dans lequel en réponse au dépassement par les mesures de la caractéristique donnée de la limite de sécurité correspondante, un indicateur visuel est affiché en association avec la certaine fenêtre de visualisation fournissant la mesure de la caractéristique donnée pour attirer l'attention de l'utilisateur sur la fenêtre de visualisation fournissant la mesure de la caractéristique donnée.

5. Procédé selon l'une quelconque des revendications précédentes, ledit procédé comprenant :
a) la réception d'un signal fournissant un identifiant utilisateur associé à l'utilisateur du système ;
b) la sélection d'une fenêtre de visualisation parmi l'ensemble possible de fenêtres de visualisation au moins en partie sur la base du signal fournissant l'identifiant utilisateur ;
c) l'affichage de la fenêtre de visualisation sélectionnée au moins en partie sur la base du signal fournissant l'identifiant utilisateur en parallèle avec la première fenêtre de visualisation et la fenêtre de visualisation supplémentaire sélectionnée.

6. Procédé selon l'une quelconque des revendications précédentes, dans lequel ledit ensemble de fenêtres de visualisation possibles comporte une fenêtre de visualisation fournissant sur un premier segment temporel les niveaux de risque associés à la progression du travail.

7. Procédé selon la revendication 6, dans lequel pour chaque sous-segment temporel dudit premier segment temporel, ledit procédé comprend le traitement des signaux fournissant les informations de progression du travail pour dériver des données fournissant les niveaux de risque respectifs associés à la progression du travail.

8. Procédé selon l'une quelconque des revendications précédentes, dans lequel au moins une fenêtre de visualisation dans ledit ensemble de fenêtres de visualisation possibles fournit des éléments d'information dérivés au moins en partie de mesures cliniques obtenues de la patiente en obstétrique.

9. Procédé selon la revendication 8, dans lequel ledit ensemble de fenêtres de visualisation possibles comporte une fenêtre de visualisation affichant des données fournissant les dilatations de col prévues au fil du temps.

10. Procédé selon l'une quelconque des revendications précédentes, dans lequel au moins une fenêtre de visualisation possible dans ledit ensemble de fenêtres de visualisation possibles fournit les mesures d'une fréquence cardiaque foetale au fil du temps.

11. Procédé selon l'une quelconque des revendications précédentes, dans lequel une fenêtre de visualisation de l'ensemble de fenêtres de visualisation possibles fournit un tracé indicatif d'une représentation de contraction utérine.

12. Procédé selon la revendication 11, dans lequel la représentation de contraction utérine est une moyenne mobile d'un comptage de contractions utérines associé à la patiente en obstétrique.

13. Procédé selon l'une quelconque des revendications précédentes, dans lequel ladite au moins une commande comporte un boîtier de sélection.

14. Procédé selon l'une quelconque des revendications 1 à 13, dans lequel ladite au moins une commande est une d'un ensemble de commandes, ledit procédé comprenant l'affichage de l'ensemble de commandes, chaque commande dans ledit ensemble de commandes étant associée à une fenêtre de visualisation respective dans l'ensemble de fenêtres de visualisation possibles, chaque commande permettant à un utilisateur de sélectionner une fenêtre de visualisation associée.

15. Procédé selon l'une quelconque des revendications précédentes, dans lequel ladite au moins une commande permet à l'utilisateur de sélectionner le sous-ensemble de fenêtres de visualisation parmi l'ensemble de fenêtres de visualisation possibles à l'aide d'un dispositif d'entrée sélectionné dans l'ensemble constitué d'une souris, d'un clavier, d'un dispositif de pointage, d'une unité de reconnaissance de la parole et d'un écran tactile.

16. Support de stockage lisible par ordinateur stockant un élément de programme conçu pour être exécuté par un processeur, ledit élément de programme mettant en oeuvre le procédé d'affichage d'informations liées au travail décrit dans l'une quelconque des revendications 1 à 15.

17. Système de serveur stockant un élément de programme pour exécution par un processeur, ledit élément de programme mettant en oeuvre le procédé d'affichage d'informations liées au travail décrit dans l'une quelconque des revendications 1 à 15.

18. Appareil mettant en oeuvre une interface utilisateur pour afficher des informations liées au travail, ledit appareil comprenant des moyens d'entrée pour recevoir des signaux fournissant des informations liées au travail et des moyens de traitement en communication avec lesdits moyens d'entrée, lesdits moyens de traitement étant conçus pour mettre en oeuvre le procédé décrit dans l'une quelconque des revendications 1 à 15.

19. Système de surveillance du travail comprenant :
a) un premier capteur destiné à recevoir un signal indicateur d'une fréquence cardiaque foetale ;
b) un second capteur destiné à recevoir un signal de contraction utérine associé à une patiente en obstétrique ;
c) un appareil mettant en oeuvre un module d'interface utilisateur graphique destiné à afficher les informations liées au travail, ledit appareil comprenant :
i) des premiers moyens d'entrée en communication avec ledit premier capteur destinés à recevoir le signal de fréquence cardiaque foetale ;
ii) des seconds moyens en communication avec ledit second capteur destinés à recevoir le signal de contraction utérine ;
iii) des moyens de traitement en communication avec lesdits premier et second moyens d'entrée ;
d) des moyens d'affichage en communication avec ledit appareil ;
e) **caractérisé en ce que** les moyens de traitement dudit appareil sont conçus pour mettre en oeuvre le procédé décrit dans l'une quelconque des revendications 1 à 15.

20. Système client-serveur mettant en oeuvre un module d'interface utilisateur graphique destiné à afficher des informations liées au travail, ledit système client-serveur comprenant un système client et un système serveur, ledit système client et ledit système serveur opérant un échange de messages sur un réseau de données, ledit système serveur stockant un élément de programme pour exécution par un processeur, **caractérisé en ce que** ledit élément de programme lors de son exécution sur ledit processeur met en oeuvre le procédé décrit dans l'une quelconque des revendications 1 à 15.

21. Système client-serveur selon la revendication 20, dans lequel le réseau de données est Internet.
